# EUROPEAN PATENT APPLICATION

(11) **EP 3 660 168 A1**
(43) Date of publication of application: **03.06.2020**
(21) Application number: 18837931.7
(22) Date of filing: 25.07.2018
(51) Int. Cl.: C12Q 1/686, C12N 15/11

(54) **METHOD FOR DETECTING MUTANT GENE**

(30) Priority: 26.07.2017 JP 2017144291
(71) Applicant: Sekisui Medical Co., Ltd., Tokyo 103-0027 (JP)
(72) Inventor: EBINUMA Hiroyuki, Tokyo 103-0027 (JP); TSUKAMOTO Yuriko, Tokyo 103-0027 (JP)
(74) Representative: Hoffmann Eitle
(86) International application number: PCT/JP2018/027878
(87) International publication number: WO 2019/022132

(57) **Abstract**

An object of the present invention is to provide a method for highly sensitively detecting, and a method for quantifying, a mutant gene (mutant allele) contained at a low frequency in a nucleic acid sample containing a wild-type allele. In one-reaction system in which a first-primers set designed to flank a mutation site of a gene of interest is mixed with a second-primers set including an ASP corresponding to the mutation, an amplification product can be obtained from a low-frequency mutant gene, and quantitativeness can be ensured, by including a competitive nucleic acid suppressing an amplification reaction from a wild-type allele of the gene, selecting a certain primer concentration condition, and controlling the cycle numbers of PCR reactions using the first and second-primers sets.

## Description

### TECHNICAL FIELD

The present invention relates to a method for detecting with high sensitivity and quantifying a mutant allele contained at a low frequency in a nucleic acid sample containing a wild-type allele.

### BACKGROUND ART

Genetic mutations include germline mutations, which are inherited genetically, and somatic mutations, which are acquired in individual cells. It has been reported that single nucleotide polymorphism (SNP), which is a germline mutation of a certain gene, and point mutation (single nucleotide mutation), which is typical somatic mutation, are implicated in various diseases, and in recent years, detection of such nucleotide sequences has been utilized for selecting patients for which a certain drug is expected to be effective.

For example, an epidermal growth factor receptor (EGFR) gene mutation test is performed as a basis for determining efficacy of a tyrosine kinase inhibitor (TKI), which is a therapeutic agent for lung cancer. Since this test is performed by using a trace amount of a cancer tissue specimen, and wild-type allelic genes derived from normal tissue and cancer tissue are mixed, the test is required to have high sensitivity and high specificity.

Among the EGFR gene mutations, a point mutation at codon 790 of exon 20 (T790M, 2369C->T) is particularly known as a resistance mutation for first-generation and second-generation TKIs such as gefitinib and afatinib. A third generation TKI (osimertinib) effective for the T790M mutation has recently been clinically used, and a test for the T790M mutation is required as a condition for application to patients who are known to have TKI resistance and experienced a recurrence of lung cancer. Furthermore, collection and examination of tissue specimens by frequent re-biopsy are required so as not to overlook signs of TKI resistance due to the T790M mutation and recurrence of lung cancer; however, highly invasive re-biopsy causes a heavy burden on patients and cannot be performed in some cases. Therefore, in recent years, osimertinib can be prescribed even when the T790M mutation is detected from cell-free DNA derived from cancer tissue flowing out into plasma. However, an amount of cell-free DNA in plasma is very small, and a mutation detection method is required to have sensitivity higher than that required for detection from a tissue specimen. Additionally, a quantitative method for capturing the changes in the amount of the T790M mutation is desired for monitoring a recurrence of lung cancer.

Reported methods of detecting gene mutations in EGFR include a real-time PCR method (Non-Patent Document 1) and an MBP-QP method (Non-Patent Document 2) achieved by combining an ASP-PCR method using an allele specific primer (ASP) with dissociation curve analysis by Q probe. The detection sensitivity of these methods is about 0.1 to 1 %, which is sufficient for a detection from tissue specimens already confirmed to contain cancer cells; however, this is considered as being insufficient for detection of low-frequency mutations in patients with recurrence. A digital PCR method having recently emerged is 100 times or more sensitive than these detection methods and enables quantification, and it is reported that using this method enables detection of mutation unable to detect with existing detection methods, such as detection of mutation from patients not treated with TKI (Non-Patent Document 3). A widely accepted idea is that even when such a low frequency T790M mutation is detected, the relationship between the proportion of mutant genes and the drug response should be evaluated in terms of the resistance to the first and second generation TKIs and the effectiveness of the third generation TKI. If this relationship is clarified, an appropriate drug can be selected in accordance with the proportion of resistance mutant gene. However, the digital PCR method enabling highly sensitive quantification is complicated in operation and requires an expensive dedicated apparatus.

The ASP-PCR method described above is a simple and relatively sensitive technique for detecting gene mutations (especially, point mutations) of EGFR, RAS, etc.; however, an improvement in specificity of ASPs is essential for constructing a highly sensitive mutation detection system with this method. ASPs are generally designed such that any one of 1 to 3 bases of the 3' end corresponds to a mutant nucleotide of a gene polymorphism such as a single nucleotide polymorphism and is further designed such that specificity is ensured by artificially adding a sequence (mismatch) not complementary to a target nucleic acid at a place other than the polymorphic site (Patent Document 1, Patent Document 2, and the unpublished patent application of the applicant on the priority date of this application (PCT/JP2017/12820)). However, ASPs having an artificially added mismatch have a lower affinity than a perfect match primer, which may cause a decrease in amplification efficiency.

For an ASP for detecting single nucleotide mutation, shortening a primer length is effective for ensuring the specificity in accordance with a difference of single base. On the other hand, in the case of amplification with an ASP having a short primer length, an annealing temperature of PCR must be reduced, which may cause concern about the occurrence of non-specific amplification. Such non-specific amplification is particularly likely to occur when multiple mutations are simultaneously amplified by using multiple primers in one-reaction system (multiplex PCR).

Nested PCR is known as a technique for reducing non-specific amplification. In this method, after a first amplification reaction is performed by a first-primers set flanking a target sequence, 1/20 to 1/50 of a first reaction solution is used as a template for a second amplification reaction to amplify an intended sequence with a second-primers set designed on the inner side of the first-primers set. This method can efficiently amplify a region encompassing the target sequence by taking advantage of the fact that even if a non-specific product is amplified due to mispriming by the first-primers set, the same non-specific region is unlikely to be amplified by the second-primers set. However, since the PCR reaction is performed twice, the operation is complicated and takes time. Moreover, since the reaction solution after the first PCR reaction is used as a template for the second PCR reaction, lids of reaction solutions containing a large amount of amplification products must be opened, which causes concern about contamination of the amplification products into the measurement environment (mutual contamination).

### CITATION LIST

### PATENT LITERATURE

Patent Document 1: Japanese Laid-Open Patent Publication No. 2005-160430
Patent Document 2: Japanese Patent No. 3937136

### NON PATENT LITERATURE

Non-Patent Document 1: Biomed Res Int. 2013; 2013: 385087.
Non-Patent Document 2: J Thorac Oncol. 2011 Oct; 6(10): 1639-48.
Non-Patent Document 3: Clin Cancer Res. 2015 Aug 1; 21(15): 3552-60.

### SUMMARY OF INVENTION

### TECHNICAL PROBLEM

To solve the conventional problems described above, an object of the present invention is to provide a method for highly sensitively detecting, and a method for quantifying, a mutant allele contained at a low frequency in a nucleic acid sample containing a wild-type allele.

### SOLUTION TO PROBLEM

The present inventors tried to perform Nested PCR in a homogeneous reaction system and found that, in one-reaction system in which a first-primers set designed to sandwich or flank a mutation site of a gene of interest is mixed with a second-primers set including an ASP corresponding to the mutation, an amplification product can be obtained from a low-frequency mutant gene highly sensitively, and quantitativeness can be ensured, by including a competitive nucleic acid suppressing an amplification reaction from a wild-type allele of the gene, selecting a certain primer concentration condition, and optionally controlling the cycle numbers of PCR reactions using the first- and second-primers sets, and thereby completing the present invention.

Therefore, the present invention provides [1] to [8] below.
[1] A method for detecting a gene mutation contained in a nucleic acid sample based on the presence or absence of an amplification product from a nucleic acid-amplification reaction using a DNA polymerase, the method comprising the steps of:
   in a nucleic acid-amplification reaction solution in which a first-primers set designed to flank a polymorphic site of the gene coexists with a second-primers set containing one or more allele specific primers for selective amplification from a nucleic acid containing the mutation,
   (a) obtaining an amplification product by amplifying a nucleic acid containing a mutant allele through a nucleic acid-amplification reaction by the first-primers set, in the presence of a competitive nucleic acid that has a sequence of a wild-type allele of the gene and that hybridizes with all or a portion of the polymorphic site, under a condition preventing a nucleic acid-amplification reaction by the second-primers set; and
   (b) causing the amplification reaction with the concentration of a primer(s) of the first-primers set reduced as compared to the concentration of primers of the second-primers set when the amplification product of step (a) is obtained; and
   (c) selectively amplifying a nucleic acid containing the mutant allele through a nucleic acid-amplification reaction under a condition allowing at least the second-primers set to act on the amplification product obtained at step (a).
[2] The method according to [1] above, wherein the condition preventing a nucleic acid-amplification reaction by the second-primers set is a temperature condition under which the allele specific primer(s) does not anneal to a nucleic acid containing the mutation.
[3] The method according to [1] or [2] above, wherein a primer of the second-primers set other than the allele specific primer(s) is designed in common with one primer of the first-primers set.
[4] The method according to any one of [1] to [3] above, wherein in the first-primers set, the concentration of a primer having the same direction as the allele specific primer(s) of the second-primers set is 1/100 to 1/20 of the concentration of the other primer(s).
[5] The method according to any one of [1] to [4] above, comprising a step of detecting the presence or absence of an amplification product of the second-primers set based on the presence or absence of a peak of the amplification product separated by ion exchange chromatography.
[6] A method for quantifying a mutant allele contained in a nucleic acid sample by using an amplification product from a nucleic acid-amplification reaction by a DNA polymerase, the method comprising the steps of:
   in a nucleic acid-amplification reaction solution in which a first-primers set designed to flank a polymorphic site of the gene coexists with a second-primers set containing one or more allele specific primers for selective amplification from a nucleic acid containing the mutation,
   (a) obtaining an amplification product by amplifying a nucleic acid containing a mutant allele through a nucleic acid-amplification reaction by the first-primers set, in the presence of a competitive nucleic acid that has a sequence of a wild-type allele of the gene and that hybridizes with all or a portion of the polymorphic site, under a condition preventing a nucleic acid-amplification reaction by the second-primers set; and
   (b) selectively amplifying, before the nucleic acid-amplification reaction by the first-primers set reaches a saturation phase when the amplification product of step (a) is obtained, a nucleic acid containing the mutant allele through a nucleic acid-amplification reaction under a condition allowing at least the second-primers set to successively act on the amplification product generated in a manner reflecting the DNA amount of the mutant allele contained in the nucleic acid sample.
[7] The method according to [6] above, wherein the cycle number of the nucleic acid-amplification reaction by the first-primers set is set to 15 to 32, wherein the cycle number of the nucleic acid-amplification reaction by the second-primers set is set to 30 to 60, and wherein the two reactions are combined and successively performed.
[8] The method according to [6] or [7] above, wherein an amplification product of the second-primers set is separated by ion exchange chromatography, and wherein the mutant allele contained in the nucleic acid sample is quantified from a peak area of the amplification product.

### ADVANTAGEOUS EFFECTS OF INVENTION

According to the present invention, a mutant gene contained at low frequency can specifically and rapidly detected and quantified. For example, even if a wild-type allele corresponding to a mutant gene (e.g., T790M) is present in large amount in a sample as in EGFR gene mutation detection in lung cancer patients, mutation can be detected and quantified with high sensitivity, which enables clinical application through risk judgment and monitoring of resistance to the first and second generation TKIs.

### BRIEF DESCRIPTION OF DRAWINGS

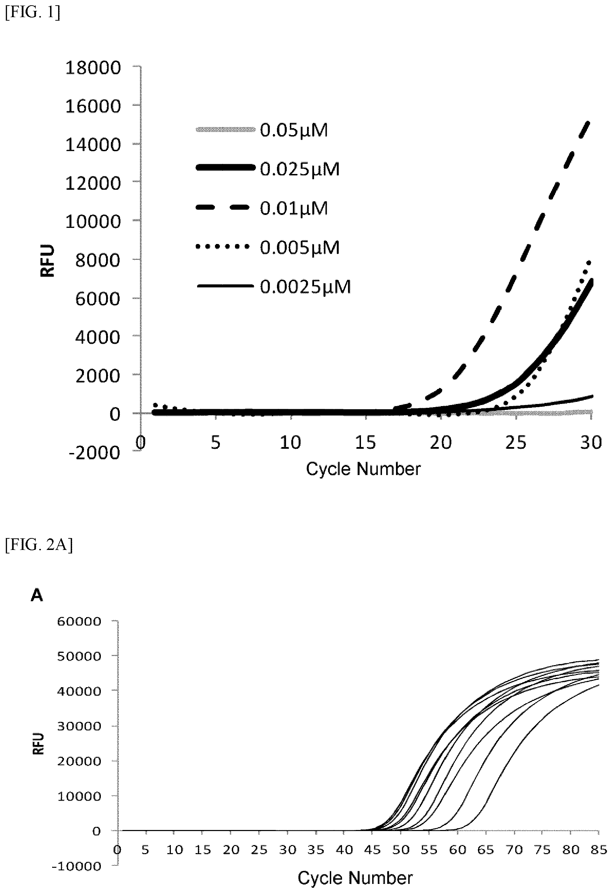

[Fig. 1] Fig. 1 shows amplification curves of 30 cycles of the second PCR reaction when PCR amplification is performed at different concentrations of a forward primer for the first PCR.

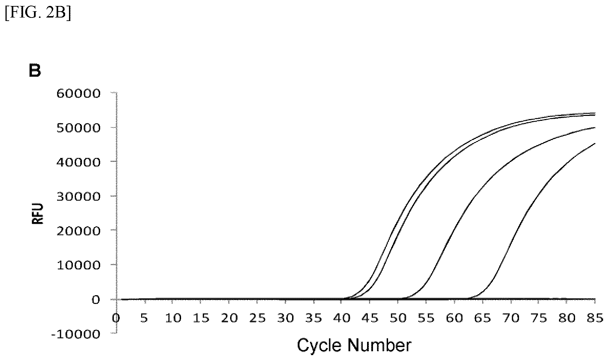

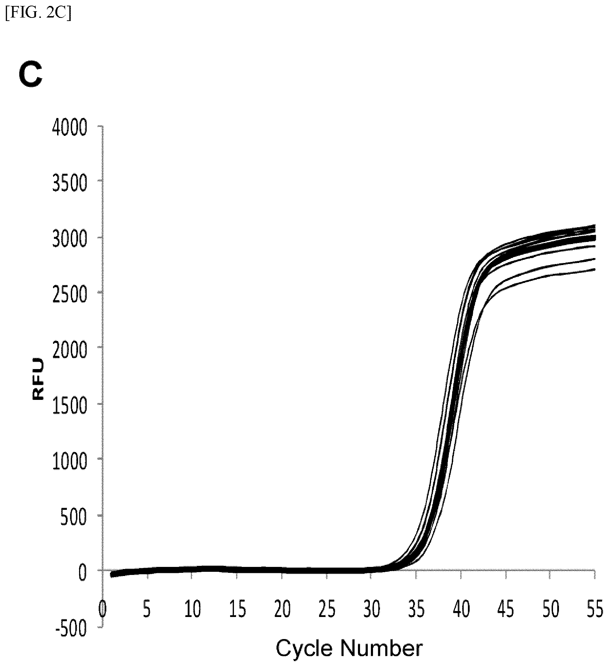

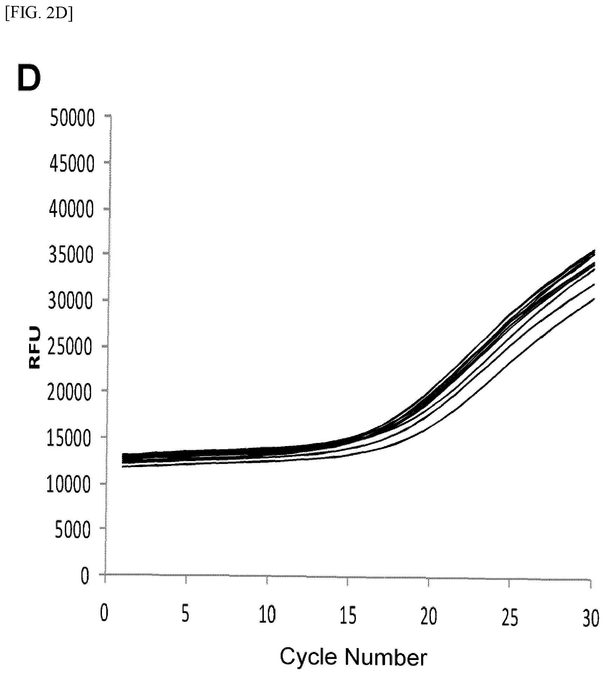

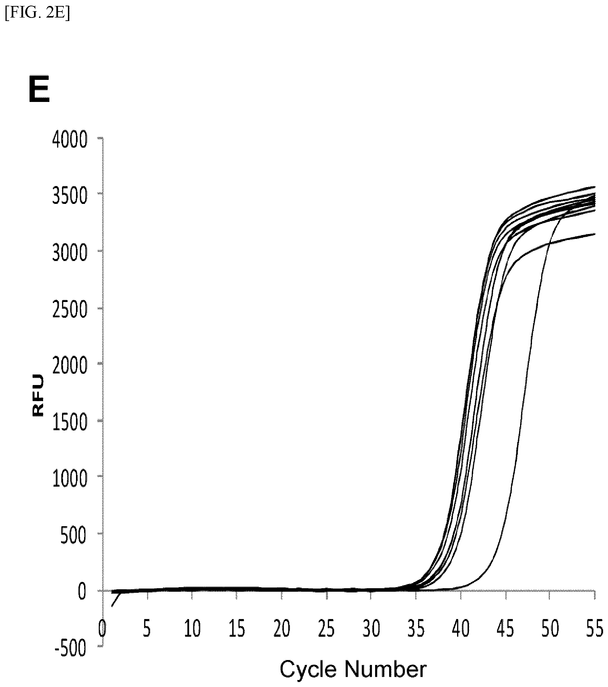

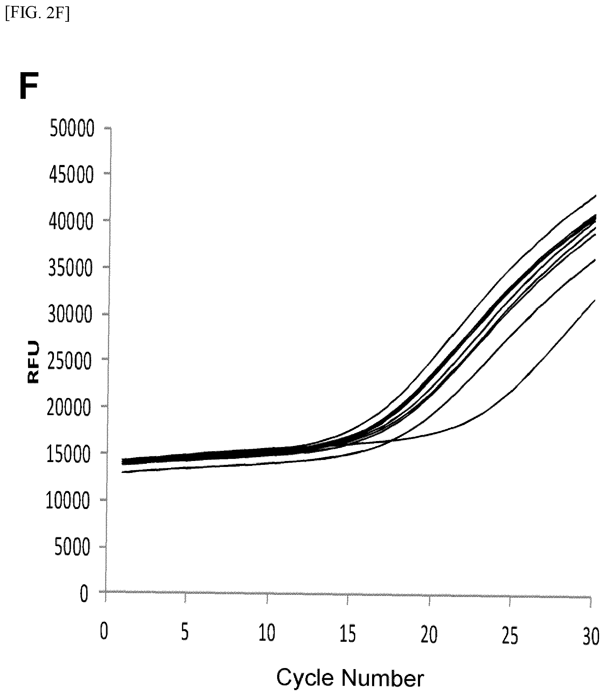

[Fig. 2] Fig. 2 shows [A] amplification curves in the case of PCR amplification from 0.05 ng of Mutant by an ASP-PCR method, [B] amplification curves in the case of PCR amplification from 0.015 ng of Mutant by the ASP-PCR method, [C] amplification curves of 55 cycles of the first PCR reaction in the case of PCR amplification performed from 0.05 ng of Mutant by using the forward primer (0.025 µM) for the first PCR, [D] amplification curves of 30 cycles of the second PCR reaction in the case of PCR amplification performed from 0.05 ng of Mutant by using the forward primer (0.025 µM) for the first PCR, [E] amplification curves of 55 cycles of the first PCR reaction in the case of PCR amplification performed from 0.015 ng of Mutant by using the forward primer (0.025 µM) for the first PCR, and [F] amplification curves of 30 cycles of the second PCR reaction in the case of PCR amplification performed from 0.015 ng of Mutant by using the forward primer (0.025 µM) for the first PCR.

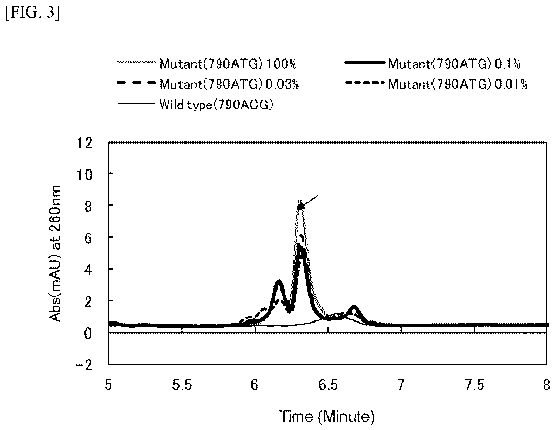

[Fig. 3] Fig. 3 shows elution peaks of amplification products obtained by ion exchange chromatography after 55 cycles of the first PCR reaction and 30 cycles of the second PCR reaction.

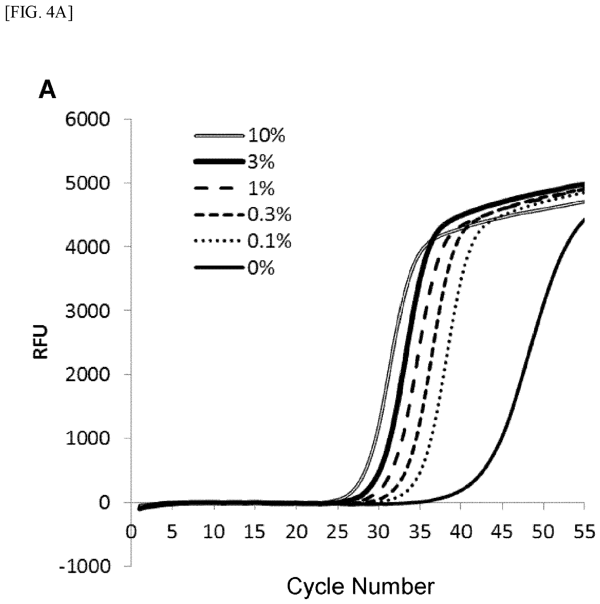

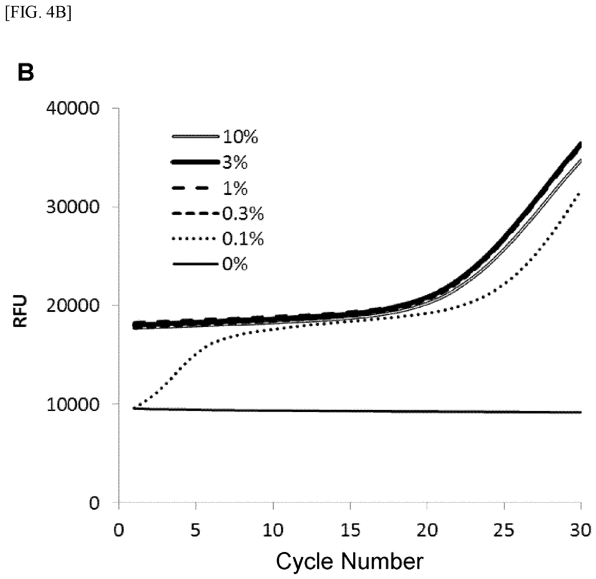

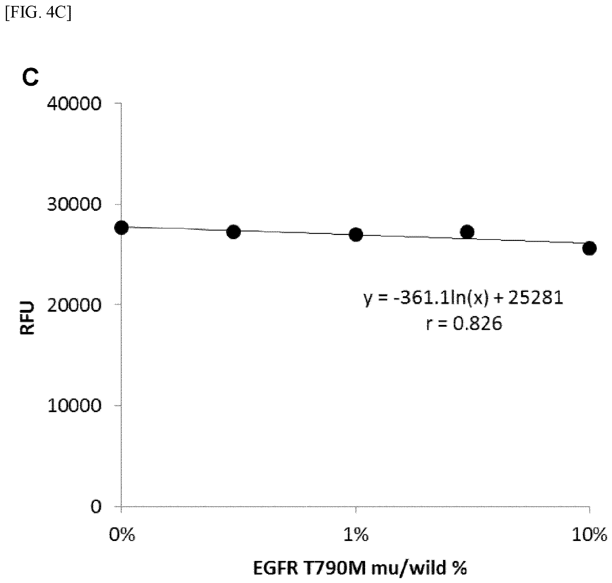

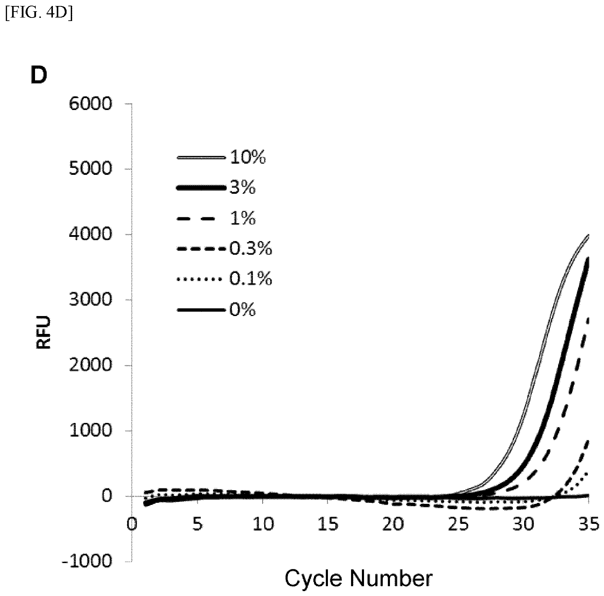

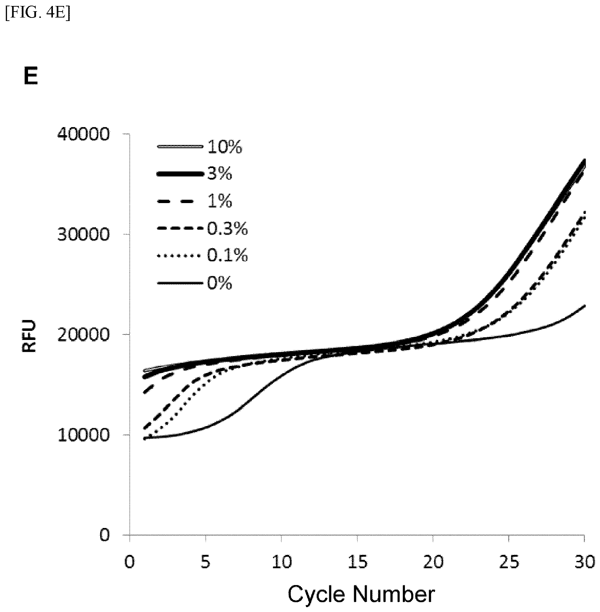

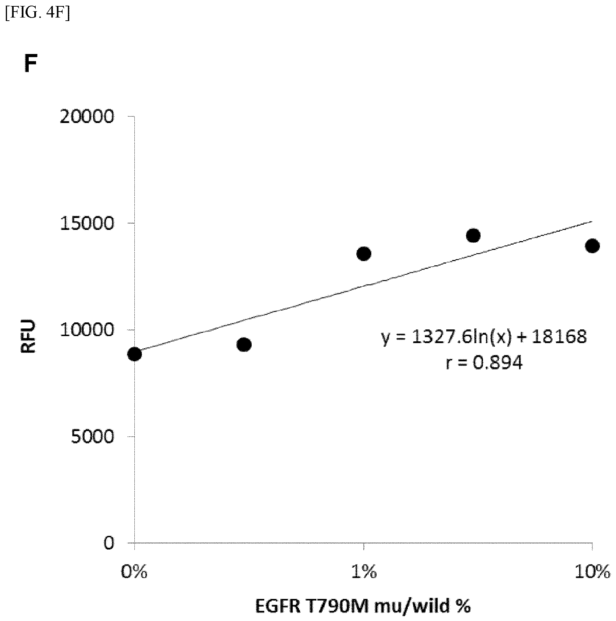

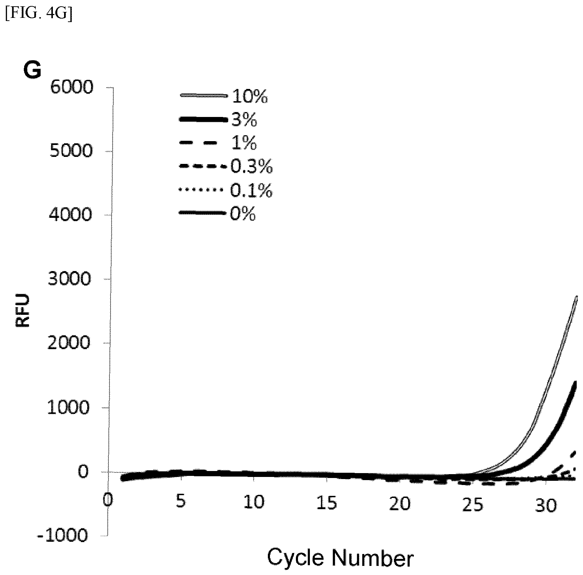

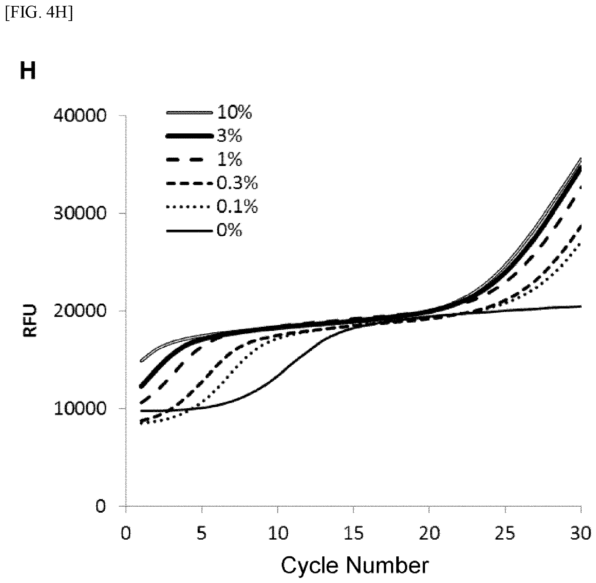

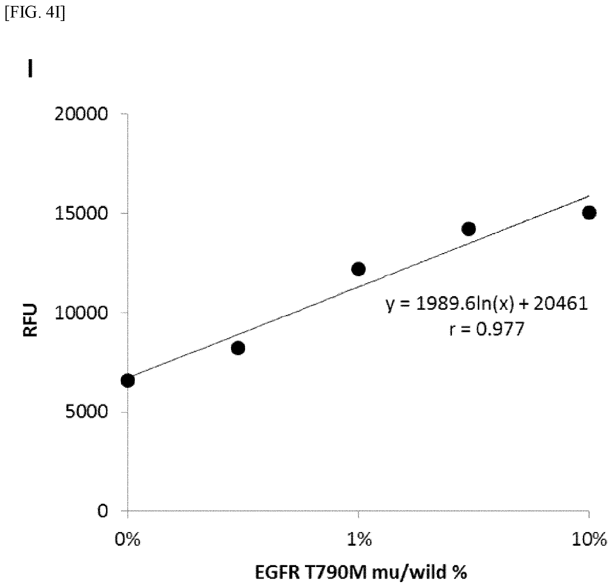

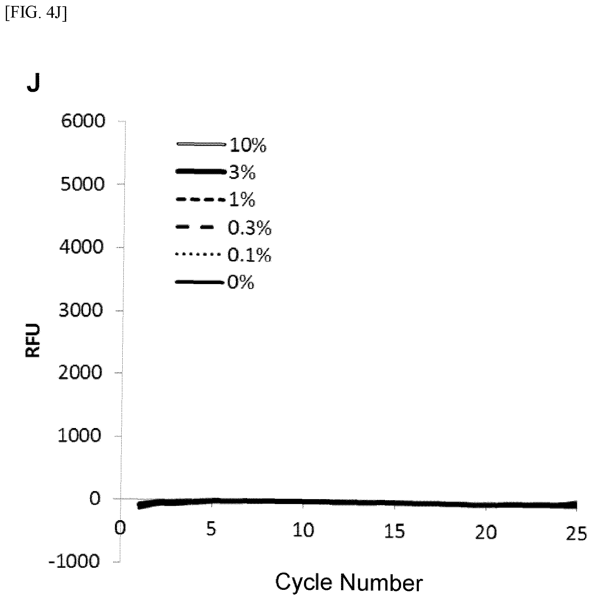

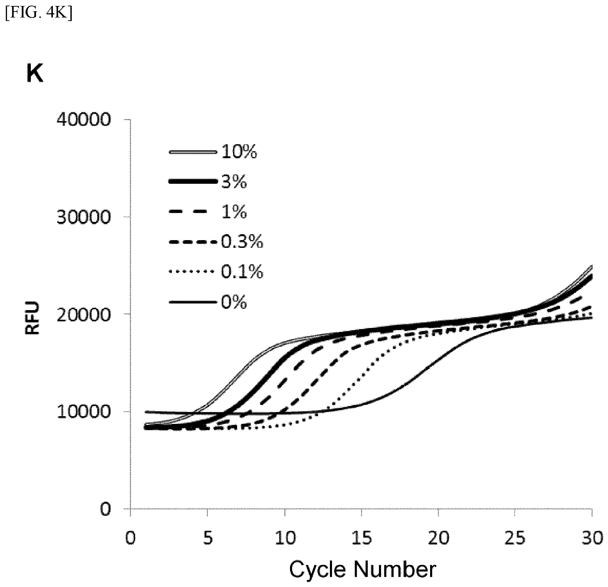

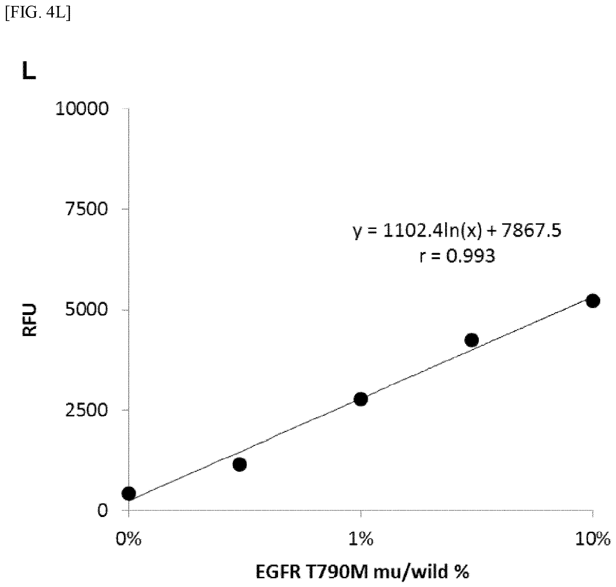

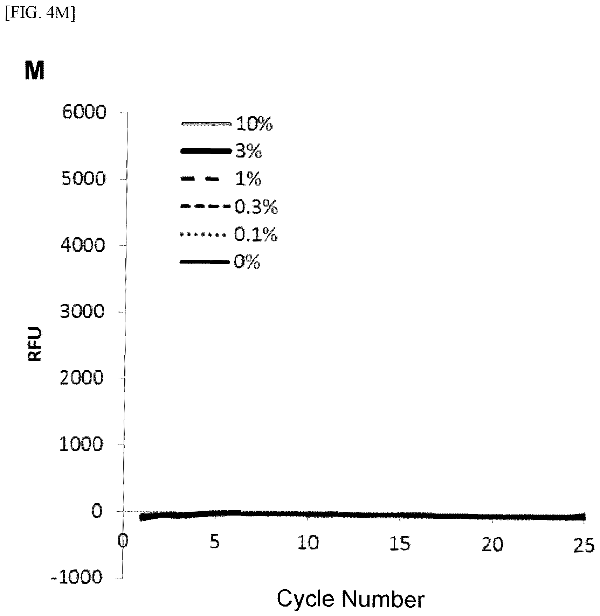

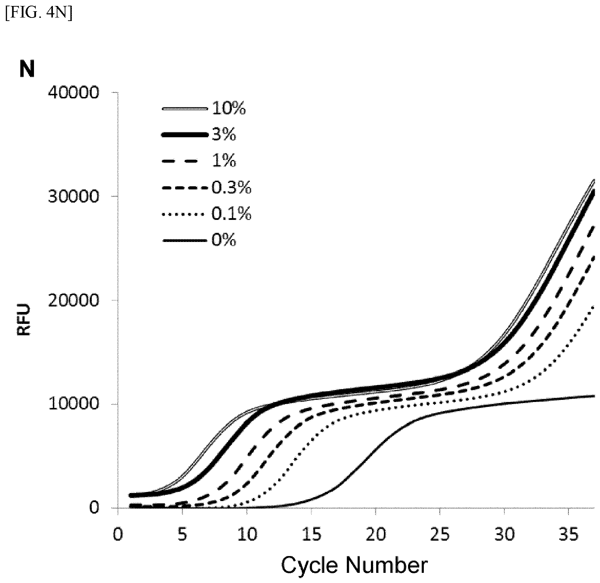

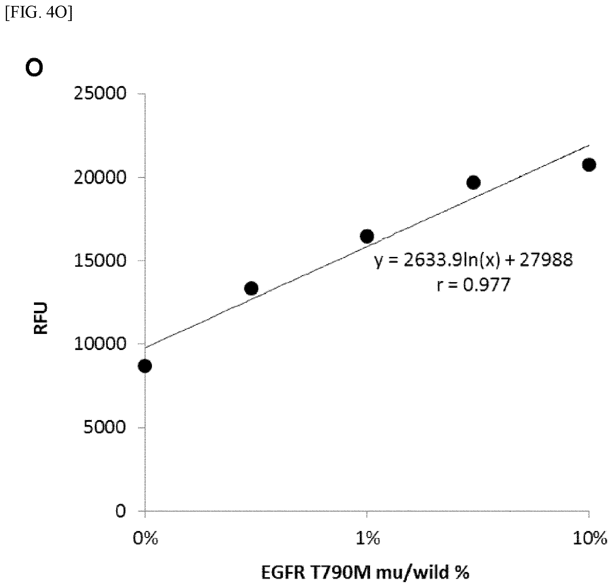

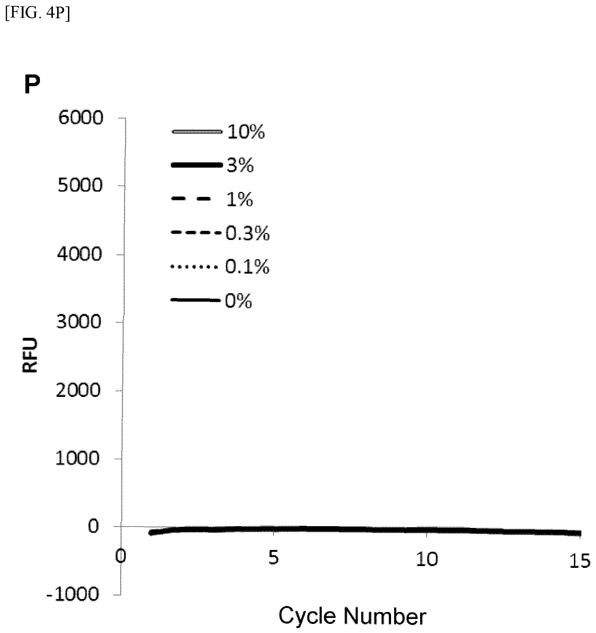

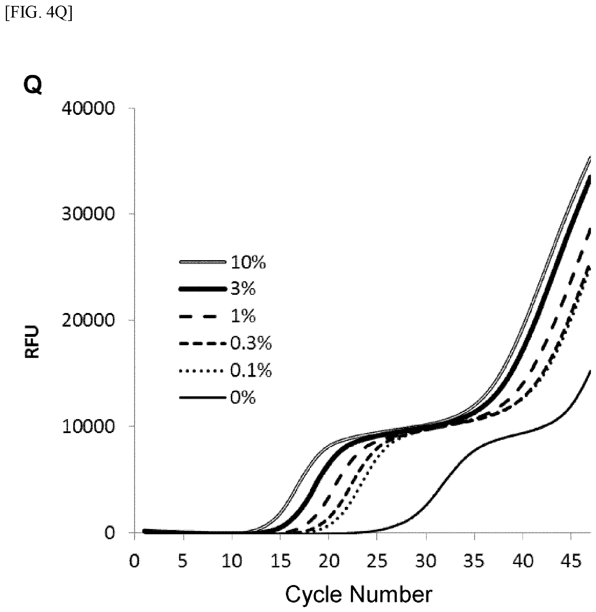

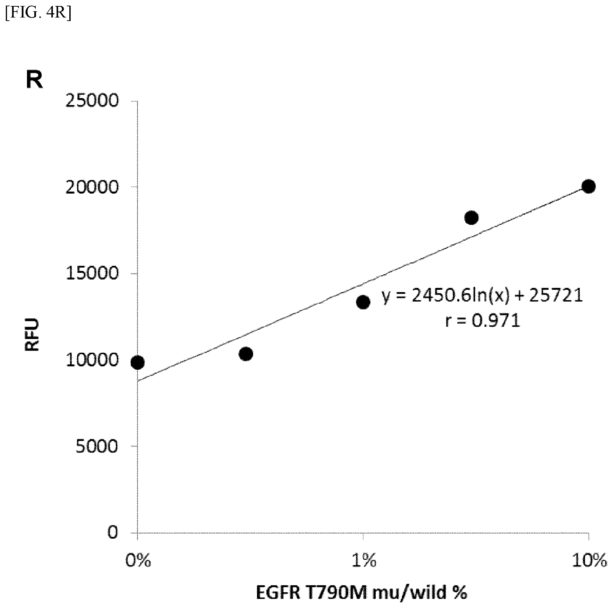

[Fig. 4] Fig. 4 shows [A] amplification curves of 55 cycles of the first PCR reaction, [B] amplification curves of 30 cycles of the second PCR reaction after 55 cycles of the first PCR reaction of [A] described above, [C] correlation between an RFU value and a T790M mutant allele proportion at the time of 30th cycle of the second PCR reaction of [B] described above, [D] amplification curves of 35 cycles of the first PCR reaction, [E] amplification curves of 30 cycles of the second PCR reaction after 35 cycles of the first PCR reaction of [D] described above, [F] correlation between the RFU value and the T790M mutant allele proportion at the time of 30th cycle of the second PCR reaction of [E] described above, [G] amplification curves of 32 cycles of the first PCR reaction, [H] amplification curves of 30 cycles of the second PCR reaction after 32 cycles of the first PCR reaction of [G] described above, [I] correlation between the RFU value and the T790M mutant allele proportion at the time of 30th cycle of the second PCR reaction of [H] described above, [J] amplification curves of 25 cycles of the first PCR reaction, [K] amplification curves of 30 cycles of the second PCR reaction after 25 cycles of the first PCR reaction of [J] described above, [L] correlation between the RFU value and the T790M mutant allele proportion at the time of 30th cycle of the second PCR reaction of [K] described above, [M] amplification curves of 25 cycles of the first PCR reaction, [N] amplification curves of 37 cycles of the second PCR reaction after 25 cycles of the first PCR reaction of [M] described above, [O] correlation between the RFU value and the T790M mutant allele proportion at the time of 37th cycle of the second PCR reaction of [N] described above, [P] amplification curves of 15 cycles of the first PCR reaction, [Q] amplification curves of 47 cycles of the second PCR reaction after 15 cycles of the first PCR reaction of [P] described above, and [R] correlation between the RFU value and the T790M mutant allele proportion at the time of 47th cycle of the second PCR reaction of [Q] described above.

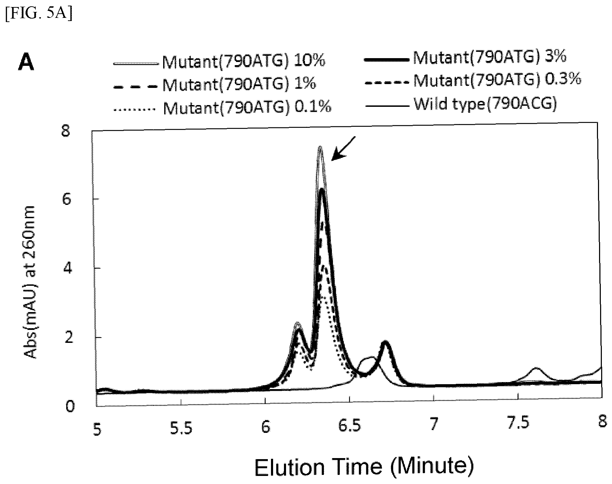

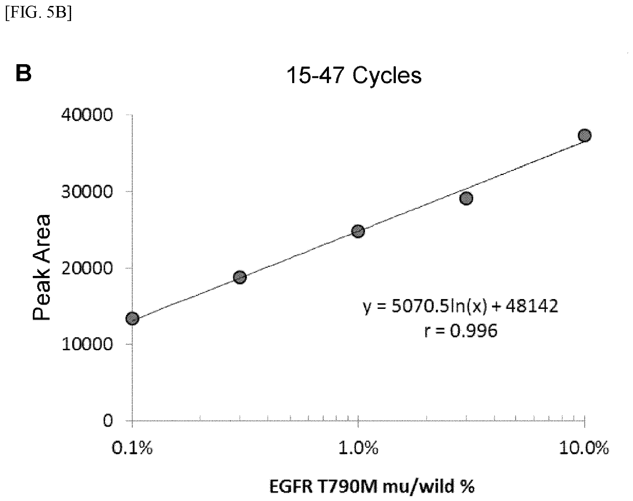

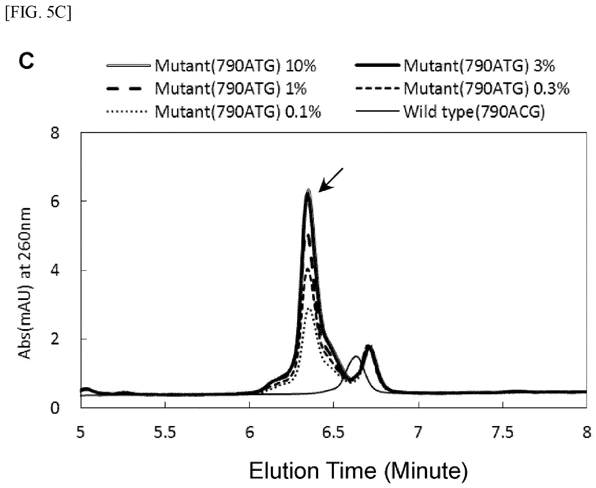

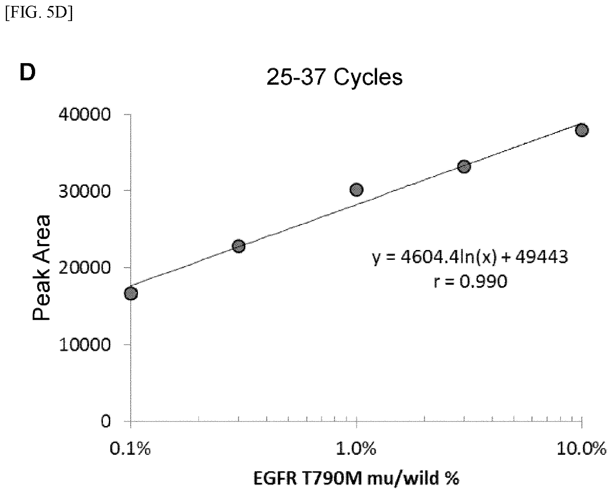

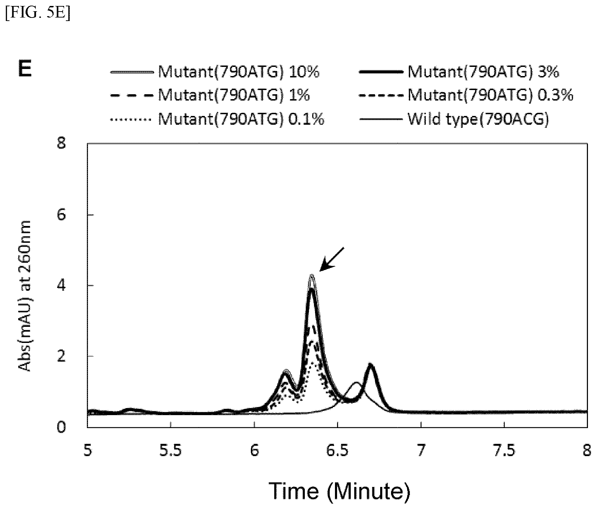

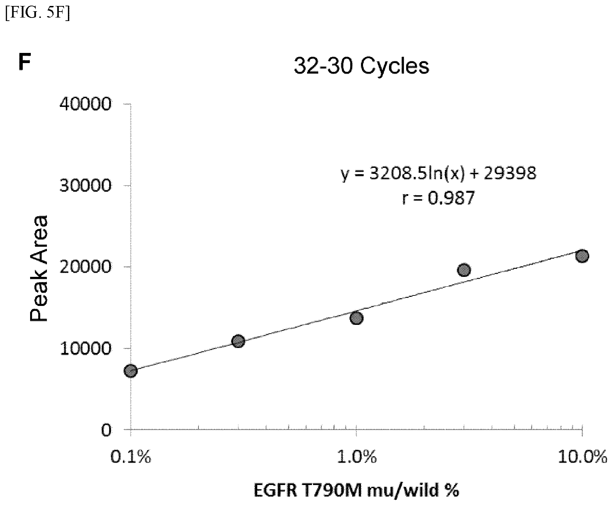

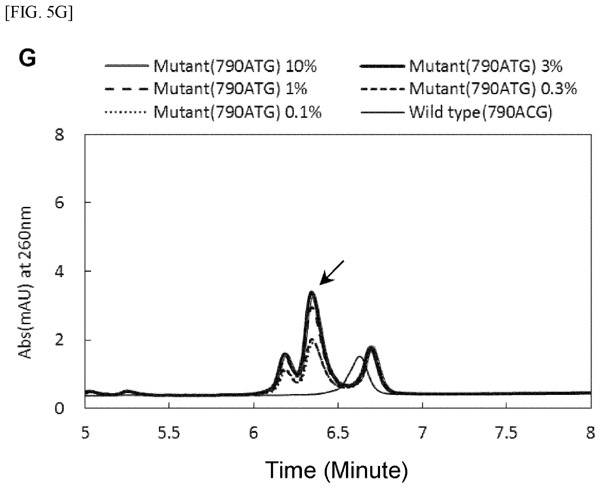

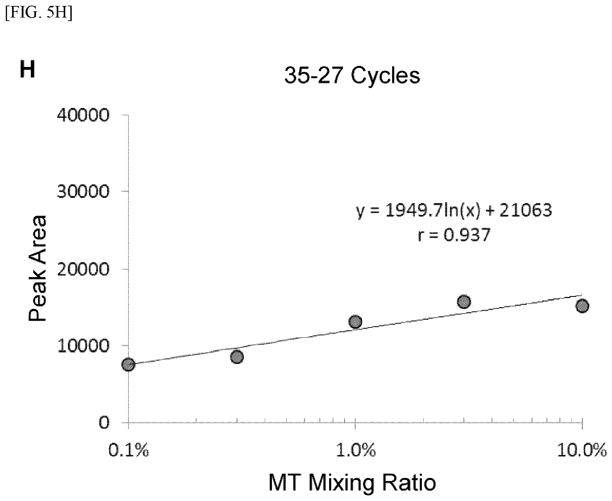

[Fig. 5] Fig. 5 shows [A] amplification product elution peaks after 15 cycles of the first PCR reaction and 47 cycles of the second PCR reaction, [B] correlation between amplification product elution peak areas of [A] described above and the T790M mutant allele proportion, [C] amplification product elution peaks after 25 cycles of the first PCR reaction and 37 cycles of the second PCR reaction, [D] correlation between amplification product elution peak areas of [C] described above and the T790M mutant allele proportion, [E] amplification product elution peaks after 32 cycles of the first PCR reaction and 30 cycles of the second PCR reaction, [F] correlation between amplification product elution peak areas of [E] described above and the T790M mutant allele proportion, [G] amplification product elution peaks after 35 cycles of the first PCR reaction and 27 cycles of the second PCR reaction, and [H] correlation between amplification product elution peak areas of [G] described above and the T790M mutant allele proportion.

### DESCRIPTION OF EMBODIMENTS

Embodiments for implementing the invention will now be described; however, the present invention is not limited to these embodiments in any way and may be implemented in various forms without departing from the spirit thereof.

In the present invention, a primer of the second-primers set other than the allele specific primer(s) (ASP) can be designed in common with one primer of the first-primers set (hereinafter, a primer having a common design will referred to as "common primer" in some cases). Therefore, the common primer plays a role in both the first-primers set and the second-primers set. In this case, the concentration of the common primer is preferably set to the concentration of the second-primers set.

In the present invention, to prevent the second-primers set coexisting during a first PCR reaction from working, a lowest Tm value of the first-primers set is designed to be higher than the lowest Tm value of the second-primers set by 10°C or more, and a difference in amplification reaction temperature is preferably 10°C or more. In the present invention, to prevent the second-primers set coexisting during the first PCR reaction from working, a condition preventing the nucleic acid-amplification reaction by the second-primers set is preferably employed.

In the present invention, the concentration of each of the primers of the first-primers set is preferably 1/100 (one-hundredth) to 1/20 (one-twentieth) of the concentration of an ASP(s) of the second-primers set. In this case, one of the primers of the first-primers set preferably has a concentration within 100 times, 20 times, or 10 times of the other primer(s), and one of the primers of the second-primers set preferably has a concentration within 100 times, 20 times, or 10 times of the other primer(s). If a primer of the second-primers set other than the ASP(s) is designed in common with the one primer of the first-primers set, the primer in the first-primers set having the same direction as the ASP(s) of the second-primers set preferably has a concentration that is 1/100 to 1/20 of the concentration of the common primer.

In the present invention, the concentration of a primer in the first-primers set having the same direction as the ASP(s) of the second-primers set is preferably 0.010 to 0.100 µM, 0.020 to 0.050 µM, or 0.025 to 0.040 µM.

In the present invention, to perform quantification of a mutant gene (measurement of a ratio to a wild-type allele and/or measurement of the copy number of the mutant gene), although any conditions for selectively amplifying a detection region including a mutant site of the mutant gene can be employed without particular limitation, a nucleic acid-amplification reaction is preferably performed by changing a condition (e.g. reducing the amplification reaction temperature) so that at least the second-primers set acts, before the nucleic acid-amplification reaction by the first-primers set reaches a saturation phase through an exponential phase. Suitably, the cycle number of the nucleic acid-amplification reaction by the first-primers set may be set to 15 to 32 times, 16 to 31 times, 17 to 30 times, 18 to 29 times, 19 to 28 times, 20 to 27 times, 21 to 26 times, 22 to 25 times, or 23 to 24 times, or 15, 16, 17, 18, 19, 20, 21, 22, 23, 24, 25, 26, 27, 28, 29, 30, 31, or 32 times; the cycle number of the nucleic acid-amplification reaction by the second-primers set may be set to 30 to 60 times, 31 to 59 times, 32 to 58 times, 33 to 57 times, 34 to 56 times, 35 to 55 times, 36 to 54 times, 37 to 53 times, 38 to 52 times, 39 to 51 times, 40 to 50 times, 41 to 49 times, 42 to 48 times, 43 to 47 times, or 44 to 46 times, or 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 times; and the two reactions may be combined and successively performed.

In this description, the phrase "the nucleic acid-amplification reaction reaches a saturation phase" means a situation which falls within at least one of the following (1) and (2): (1) the amount of the amplification product after the Nth cycle of the nucleic acid-amplification reaction is equal to or less than 1.3 times the amount of the amplification product after the N-1th cycle of the nucleic acid-amplification reaction; and (2) an inflection point appears on a curve obtained by plotting the cycle number of the nucleic acid-amplification reaction as the x-axis and the amount of the amplification product after the corresponding cycle of the nucleic acid-amplification reaction as the y-axis (when second order differential coefficient is positive).

In the present invention, the chain length of the first amplification product is preferably 250 bp or less and 50 bp or more. In the present invention, when cell-free DNA in blood is detected, the chain length of the first amplification product is preferably 120 bp or less and 50 bp or more.

In the present invention, examples of a competitive nucleic acid include a peptide nucleic acid (PNA), a locked nucleic acid (LNA), and an oligonucleotide with a 3' end subjected to modification such as phosphorylation so that a DNA synthesis reaction from the 3' end by DNA polymerase does not occur. The concentration of the competitive nucleic acid is preferably a concentration that suppresses amplification of a wild-type allele, which is not an object of detection, and that does not inhibit amplification of a mutant gene to be detected in the amplification by the first-primers set. More specifically, the concentration of the competitive nucleic acid is 0.01 to 1.00 µM, 0.01 to 0.75 µM, 0.02 to 0.50 µM, 0.03 to 0.30 µM, 0.04 to 0.25 µM, 0.05 to 0.20 µM, 0.06 to 0.17 µM, 0.07 to 0.14 µM, 0.08 to 0.13 µM, and 0.09 to 0.11 µM.

In the present invention, the sequence of the competitive nucleic acid is completely identical to the sequence of at least 10 nucleotides, preferably 10 to 30 nucleotides, 11 to 29 nucleotides, 12 to 28 nucleotides, 13 to 27 nucleotides, 14 to 26 nucleotides, 15 to 25 nucleotides, 16 to 24 nucleotides, 17 to 23 nucleotides, 18 to 22 nucleotides, or 19 to 21 nucleotides, or 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides, 21 nucleotides, 22 nucleotides, 23 nucleotides, 24 nucleotides, 25 nucleotides, 26 nucleotides, 27 nucleotides, 28 nucleotides, 29 nucleotides, or 30 nucleotides including a polymorphic site of a wild type allele of the gene to be subjected to mutation detection. In the present invention, the sequence of the competitive nucleic acid to be employed may be either of the two strands of DNA of the gene to be subjected to mutation detection. In the present invention, the length of the competitive nucleic acid is preferably 10 to 40 nucleotides, 11 to 39 nucleotides, 12 to 38 nucleotides, 13 to 37 nucleotides, 14 to 36 nucleotides, 15 to 35 nucleotides, 16 to 34 nucleotides, 17 to 33 nucleotides, 18 to 32 nucleotides, 19 to 31 nucleotides, 20 to 30 nucleotides, 21 to 29 nucleotides, 22 to 28 nucleotides, 23 to 27 nucleotides, 24 to 26 nucleotides, 25 to 25 nucleotides, 26 to 34 nucleotides, 27 to 33 nucleotides, 28 to 32 nucleotides, or 29 to 31 nucleotides, or 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides, 21 nucleotides, 22 nucleotides, 23 nucleotides, 24 nucleotides, 25 nucleotides, 26 nucleotides 27 nucleotides, 28 nucleotides, 29 nucleotides, 30 nucleotides, 31 nucleotides, 32 nucleotides, 33 nucleotides, 34 nucleotides, 35 nucleotides, 36 nucleotides, 37 nucleotides, 38 nucleotides, 39 nucleotides, 40 nucleotides, 41 nucleotides, 42 nucleotides, 43 nucleotides, 44 nucleotides, 45 nucleotides, 46 nucleotides, 47 nucleotides, 48 nucleotides, 49 nucleotides, or 50 nucleotides. In the present invention, the position of the polymorphic site in the competitive nucleic acid may be any position as long as the polymorphic site is entirely or partially included in the sequence of the competitive nucleic acid. If the polymorphic site is entirely included in the sequence of the competitive nucleic acid, in an embodiment, the polymorphic site preferably corresponds to a nucleotide(s) from 1/10 to 9/10 of the full length from the 5' side. In this case, for example, if the competitive nucleic acid has the length of 49 nucleotides, the polymorphic site preferably corresponds to a nucleotide(s) from 4.9 (=49×1/10) to 44.1, i.e., from fifth to 44th nucleotides. In another embodiment, the polymorphic site preferably corresponds to a nucleotide(s) from 2/10 to 8/10, a nucleotide(s) from 3/10 to 7/10, or a nucleotide(s) from 4/10 to 6/10, of the full length from the 5' side.

In the present invention, a method for detecting or quantifying the amplification product of the second-primers set is preferably a method for measuring a generated fluorescence intensity with an intercalator such as SYBR (registered trademark) Green, and a more preferable method is a method in which an amplification product is separated by ion exchange chromatography and is detected or quantified through the presence or absence of a peak thereof and the comparison of the peak area. While the peak of the amplification product is usually detected by absorbance measurement at 260 nm, using an ASP labeled with a fluorescent dye at the 5' end enables detection of the amplification product with a fluorescence detector and is useful when the amplification product is distinguished from a non-specific amplification product other than the ASP. Examples of the fluorescent dye used in such a case include the Alexa Fluor (registered trademark) series, the Cy (registered trademark) series, the ATTO series, the DY series, the DyLight (registered trademark) series, FAM, TAMRA, etc.

In the present invention, a nucleic acid sample is preferably a sample in which a wild-type allele and a mutant allele are mixed, and particularly preferably a sample in which a mutant allele is mixed or suspected to be mixed with a wild-type allele at a low frequency. In this description, "low frequency" means that a mixing ratio (% of mu/wild) of wild-type allele DNA (wild) and mutant allele DNA (mu) in a sample is 0.001 to 0.01 %, 0.001 to 0.02 %, 0.001 to 0.05 %, 0.001 to 0.1 %, 0.001 to 0.2 %, 0.001 to 0.5 %, 0.001 to 1 %, 0.001 to 2 %, 0.001 to 5 %, 0.001 to 10 %, 0.002 to 0.01 %, 0.002 to 0.02 %, 0.002 to 0.05 %, 0.002 to 0.1 %, 0.002 to 0.2 %, 0.002 to 0.5 %, 0.002 to 1 %, 0.002 to 2 %, 0.002 to 5 %, 0.002 to 10 %, 0.005 to 0.01 %, 0.005 to 0.02 %, 0.005 to 0.05 %, 0.005 to 0.1 %, 0.005 to 0.2 %, 0.005 to 0.5 %, 0.005 to 1 %, 0.005 to 2 %, 0.005 to 5 %, 0.005 to 10 %, 0.01 to 0.01 %, 0.01 to 0.02 %, 0.01 to 0.05 %, 0.01 to 0.1 %, 0.01 to 0.2 %, 0.01 to 0.5 %, 0.01 to 1 %, 0.01 to 2 %, 0.01 to 5 %, or 0.01 to 10 %, or 0.001 %, 0.002 %, 0.005 %, 0.01 %, 0.02 %, 0.05 %, 0.1 %, 0.2 %, 0.5 %, 1 %, 2 %, 5 %, or 10 %. The sample is preferably a biological sample derived from a cancer tissue of a cancer patient having been sensitive to a tyrosine kinase inhibitor (TKI). In this description, "high sensitivity" means that a gene mutation contained in a nucleic acid sample can be detected when the DNA concentration of the mutant allele in the sample is 0.0001 to 0.001 ng/µL, 0.0001 to 0.003 ng/µL, 0.0001 to 0.01 ng/µL, 0.0001 to 0.03 ng/µL, 0.0001 to 0.1 ng/µL, 0.0001 to 0.3 ng/µL, 0.0001 to 1 ng/µL, 0.0003 to 0.001 ng/µL, 0.0003 to 0.003 ng/µL, 0.0003 to 0.01 ng/µL, 0.0003 to 0.03 ng/µL, 0.0003 to 0.1 ng/µL, 0.0003 to 0.3 ng/µL, 0.0003 to 1 ng/µL, 0.001 to 0.003 ng/µL, 0.001 to 0.01 ng/µL, 0.001 to 0.03 ng/µL, 0.001 to 0.1 ng/µL, 0.001 to 0.3 ng/µL, 0.001 to 1 ng/µL, 0.003 to 0.01 ng/µL, 0.003 to 0.03 ng/µL, 0.003 to 0.1 ng/µL, 0.003 to 0.3 ng/µL, 0.003 to 1 ng/µL, 0.01 to 0.03 ng/µL, 0.01 to 0.1 ng/µL, 0.01 to 0.3 ng/µL, 0.01 to 1 ng/µL, 0.03 to 0.1 ng/µL, 0.03 to 0.3 ng/µL, or 0.03 to 1 ng/µL, or 0.0001 ng/µL, 0.0003 ng/µL, 0.001 ng/µL, 0.003 ng/µL, 0.01 ng/µL, 0.03 ng/µL, 0.1 ng/µL, 0.3 ng/µL, or 1 ng/µL,

In the present invention, the gene mutation is preferably a point mutation at codon 790 of exon 20 of the EGFR gene (T790M, 2369C->T). Sequences around codon 790 of exon 20 of the EGFR gene are shown as SEQ ID NO: 1 (wild-type) and SEQ ID NO: 2 (mutant).
(EGFR Gene T790M Wild Sequence [ACG] Fragment) 
[Chem 1]

ACTCAAGATCGCATTCATGCGTCTTCACCTGGAAGGGGTCCATGTGCCCCTCCTTCTGGC
CACCATGCGAAGCCACACTGACGTGCCTCTCCCTCCCTCCAGGAAGCCTACGTGATGGCC
AGCGTGGACAACCCCCACGTGTGCCGCCTGCTGGGCATCTGCCTCACCTCCACCGTGCAG
CTCATCACGCAGCTCATGCCCTTCGGCTGCCTCCTGGACTATGTCCGGGAACACAAAGAC
AATATTGGCTCCCAGTACCTGCTCAACTGGTGTGTGCAGATCGCAAAGGTAATCAGGGAA
GGGAGATACGGGGAGGGGAGATAAGGAGCCAGGATCCTCACATGCGGTCTGCGCTCCTGG
(EGFR Gene T790M Mutant Sequence [ATG] Fragment)

[Chem 2]

ACTCAAGATCGCATTCATGCGTCTTCACCTGGAAGGGGTCCATGTGCCCCTCCTTCTGGC
CACCATGCGAAGCCACACTGACGTGCCTCTCCCTCCCTCCAGGAAGCCTACGTGATGGCC
AGCGTGGACAACCCCCACGTGTGCCGCCTGCTGGGCATCTGCCTCACCTCCACCGTGCAG
CTCATCATGCAGCTCATGCCCTTCGGCTGCCTCCTGGACTATGTCCGGGAACACAAAGAC
AATATTGGCTCCCAGTACCTGCTCAACTGGTGTGTGCAGATCGCAAAGGTAATCAGGGAA
GGGAGATACGGGGAGGGGAGATAAGGAGCCAGGATCCTCACATGCGGTCTGCGCTCCTGG

In the present invention, the nucleic acid-amplification reaction is preferably a polymerase chain reaction method.

In the present invention, the allele specific primer is preferably a primer that has a base of the second nucleotide from the 3' end corresponding to the base of the mutant nucleotide of the mutation, a base of the third nucleotide from the 3' end not complementary to the base of the corresponding nucleotide of the target nucleic acid, and bases of the other nucleotides complementary to the bases of the corresponding nucleotides of the target nucleic acid. In another form of the present invention, the allele specific primer is preferably a primer that has a base of the third nucleotide from the 3' end corresponding to the base of the mutant nucleotide of the mutation, a base of the second nucleotide from the 3' end not complementary to the base of the corresponding nucleotide of the target nucleic acid, and the bases of the other nucleotides complementary to the bases of the corresponding nucleotides of the target nucleic acid.

In the present invention, when we say that the first-primers set and the second-primers set coexist, this means that all the primers included in the first-primers set and the second-primers set are present in a single continuous liquid phase (i.e., in a nucleic acid-amplification reaction solution).

In the present invention, the condition preventing the nucleic acid-amplification reaction by the second-primers set is preferably achieved by using a temperature condition under which the allele specific primer does not anneal to a nucleic acid containing the mutation. More specifically, the condition preventing the nucleic acid-amplification reaction by the second-primers set is preferably achieved by using a temperature condition 1 to 19°C higher, 2 to 18°C higher, 3 to 17°C higher, 4 to 16°C higher, 5 to 15°C higher, 6 to 14°C higher, 7 to 13°C higher, 8 to 12°C higher, or 9 to 11°C higher, or 1°C higher, 2°C higher, 3°C higher, 4°C higher, 5°C higher, 6°C higher, 7°C higher, 8°C higher, 9°C higher, 10°C higher, 11°C higher, 12°C higher, 13°C higher, 14°C higher, 15°C higher, 16°C higher, 17°C higher, 18°C higher, 19°C higher, 20°C higher, than the melting temperature (Tm) of the allele specific primer.

In the present invention, the polymorphic site is preferably a site including a single nucleotide polymorphism. In the polymorphic site of interest of the gene of interest, an allele with mutation is referred to as a mutant allele, and an allele without mutation is referred to as a wild-type allele.

In the present invention, a wild-type allele means an allele having no mutation to be detected at the mutation site of a gene mutation to be detected. In the present invention, a wild-type allele is preferably an allele in which the amino acid at codon 790 of exon 20 of the EGFR gene is T.

In the present invention, a mutant allele means an allele having a mutation to be detected at the mutation site of a gene mutation to be detected. In the present invention, a mutant allele is preferably an allele in which the amino acid at codon 790 of exon 20 of the EGFR gene is M.

In the present invention, a method for quantifying a mutant allele in a nucleic acid sample is preferably a method in which the amplification product of the second-primers set is separated by ion exchange chromatography so as to quantify the mutant allele contained in the nucleic acid sample from the peak area of the amplification product in a curve represented by an elution time on the x-axis and an amount of eluted DNA on the y-axis. In this case, the peak area of the amplification product can be obtained as, for example, an area of a region surrounded by the curve and a baseline that is a straight line connecting minimum points (least points if no minimum point exists) on both sides of the peak. By using a nucleic acid sample containing a known amount of the mutant allele as a control sample and making a comparison with the peak area of the amplification product obtained for the control sample, the absolute amount of the mutant allele contained in the nucleic acid sample of interest can be quantified.

### EXAMPLES

The present invention will hereinafter be described in detail with examples; however, the present invention is not limited to the following examples.

### [Example 1]

### Highly-Sensitive Detection of T790M Mutant Allele of EGFR Gene Exon 20 (1)

By using a DNA extracted and purified from the NCI-H1975 cell line as a DNA with T790M mutation of exon 20 of the human EGFR gene and using a DNA extracted and purified from the K562 cell line as a DNA without the mutation, a concentration range of the first-primers set ensuring performance of a measurement system designed according to the present invention was evaluated.

### • Primers

The first-primers set (SEQ ID NOS: 3 and 4) used for the first PCR reaction was set in regions flanking the T790M mutation, and the reverse primer thereof is designed as a common primer with the reverse primer of the second-primers set (SEQ ID NOS: 4 and 5). A forward primer of the second-primers set is an allele specific primer corresponding to the T790M (2369C->T) mutation and has a base of the second nucleotide from the 3' end corresponding to a base (T) of the mutant nucleotide of the mutation, a base (T) of the third nucleotide from the 3' end not complementary to the base of the corresponding nucleotide of the target nucleic acid, and bases of the other nucleotides complementary to the bases of the corresponding nucleotides of the target nucleic acid. The amplification product obtained by the combination of the primer of SEQ ID NO: 3 and the primer of SEQ ID NO: 4 has a chain length of 79 bp, and the amplification product obtained by the primer of SEQ ID NO: 4 and the primer of SEQ ID NO: 5 has a chain length of 65 bp.
SEQ ID NO: 3 (first forward primer)
   [Chem 3] 5'-TGCCTCACCTCCACCGTGC-3'
SEQ ID NO: 4 (first/second reverse primer)
   [Chem 4] 5'-CTTTGTGTTCCCGGACATAGTC-3'
SEQ ID NO: 5 (second forward primer)
   [Chem 5] 5'-CGTGCATCTCATCTTG-3'

### • Competitive Nucleic Acid

To suppress amplification of DNA containing the wild-type sequence of codon 790 of EGFR exon 20, PNA was applied as a competitive nucleic acid having a sequence complementary to the codon 790 wild-type sequence. The synthesis of PNA having the base sequence shown as SEQ ID NO: 6 was commissioned to a competitive nucleic acid synthesis commissioned company (Panagene).

SEQ ID NO: 6 (competitive nucleic acid; PNA)
[Chem 6] N'-GCTCATCACGCAGCTCATGC-C'

### • Preparation of Template DNA

A template DNA of this example was prepared by mixing a DNA (mutant; mu) extracted from the NCI-H1975 cell line and a DNA (wild) extracted from the K562 cell line in respective proportions so that the total concentration was 30 ng/µL. The wild-derived DNA and the mutant-derived DNA were mixed at a mixing ratio (% of mu/wild) of 0.01 % and 0 % (control) for respective preparations.

### • Reagents

Twenty five (25) µL of a reaction solution containing the following reagents was prepared and amplification was performed by using a thermal cycler device CFX96 (Bio-Rad).

**[Table 1]**

| | |
|---|---|
| 5×Buffer (for Q5) | 5µL |
| 10mM dNTP | 0.5µL |
| 0.05 to 1µM first forward primer (SEQ ID NO:3) | 1.25µL |
| 10µM first/second reverse primer (SEQ ID NO:4) | 1.25µL |
| 10µM second forward primer (SEQ ID NO:5) | 125µL |
| 1 µM competitive nucleic acid (SEQ ID NO:6) | 2.5µL |
| 20×EvaGreen | 1.25µL |
| 2000U/mL Q5 DNA polymerase | 0.25µL |
| Nuclease-free Water | 6.75µL |
| DNA specimen | 5µL |

### • Amplification Conditions

The amplification was performed under the following conditions.
98°C: 30 seconds
First PCR reaction; 98°C: 10 seconds, 78°C: 15 seconds (55 cycles)
Second PCR reaction; 98°C: 10 seconds, 56°C: 15 seconds (30 cycles)

Fig. 1 shows amplification curves of the second PCR reaction (mixing ratio: 0.01 %) when the amplification reaction was performed while the first forward primer having the same direction as the allele specific primer of the second-primers set is reduced in concentration. In the case that the reverse primer concentration for the first PCR was 0.5µM, an amplification in the second PCR reaction was recognized when the forward primer concentration for the first PCR was within a range of 0.005 to 0.025µM, i.e., the concentration of the primer in the first-primers set having the same direction as the ASP of the second-primers set was 1/100 to 1/20 of the other primer. The detection method of the present invention enabled the amplification even at a very low mutant gene mixing rate of 0.01 %.

### [Example 2]

### Highly-Sensitive Detection of T790M Mutant Allele of EGFR Gene Exon 20 (2)

By using a DNA extracted and purified from the NCI-H1975 cell line as a DNA with T790M mutation of exon 20 of the human EGFR gene, the measurement sensitivity of a measurement system designed according to the present invention was compared with an ASP-PCR method of a conventional technique not including the forward primer for the first PCR.

### • Preparation of Template DNA

A template for this example was prepared and used such that the concentration of the DNA (Mutant) extracted from the NCI-H1975 cell line was 0.01 ng/µL and 0.003 ng/µL.

### • Reagents

Twenty five (25) µL of a reaction solution containing the following reagents was prepared and amplification was performed by using a thermal cycler device CFX96 (Bio-Rad).

### (1) ASP-PCR Method (Conventional Technique)

**[Table 2]**

| | |
|---|---|
| 5×Buffer (for Q5) | 5µL |
| 10mM dNTP | 0.5µL |
| 10µM first/second reverse primer (SEQ ID NO:4) | 1.25µL |
| 10µM second forward primer (SEQ ID NO:5) | 1.25µL |
| 20×EvaGreen | 1.25µL |
| 2000U/mL Q5 DNA polymerase | 0.25µL |
| Nucl ease-free Water | 10.5µL |
| DNA specimen | 5µL |

### (2) In the Case Where First PCR Forward Primer Is Used (Present Invention)

**[Table 3]**

| | |
|---|---|
| 5×Buffer (for Q5) | 5µL |
| 10mM dNTP | 0.5µL |
| 0.5µMfirst forward primer (SEQ ID NO:3) | 1.25µL |
| 10µM first/second reverse primer (SEQ ID NO:4) | 1.25µL |
| 10µM second forward primer (SEQ ID NO:5) | 1.25µL |
| 20×EvaGreen | 1.25µL |
| 2000U/mL Q5 DNA polymerase | 0.25µL |
| Nuclease-free Water | 9.25µL |
| DNA specimen | 5µL |

### • Amplification Conditions

### (1) ASP-PCR Method (Conventional Technique)

98°C: 30 seconds
PCR reaction; 98°C: 10 seconds, 56°C: 15 seconds (85 cycles)

### (2) In the Case Where First PCR Forward Primer Is Used (Present Invention)

The conditions were the same as Example 1.

Figs. 2[A] and 2[B] show amplification curves when an amplification reaction was performed by the ASP-PCR method without using the first PCR forward primer. In the case of the DNA amount of 0.05 ng/reaction [A], amplification was recognized in all of 10 measurements; however, the cycle number at the rise of the amplification curves varied widely from 45 to 60 cycles. In the case of the small DNA amount of 0.015 ng/reaction [B], no amplification was observed in 6 out of 10 measurements. Even in the four measurements in which the amplification was recognized, the cycle number varied at the rise of the amplification curves, and the reproducibility was poor.

On the other hand, Figs. 2[C] to 2[F] show amplification curves of a first reaction and a second reaction when an amplification reaction was performed by using the forward primer for the first PCR at the concentration of 0.025 µM, which is 1/20 of the reverse primer for the first PCR. In the case of the DNA amount of 0.05 ng/reaction ([C] and [D]), amplification was recognized in all of 10 measurements, and the cycle number at the rise of the amplification curves was uniform, which indicates that the amplification can be performed with good reproducibility. Even in the case of the small DNA amount of 0.015 ng/reaction ([E] and [F]), amplification was recognized in all of 10 measurements. Although the cycle number at the rise of the amplification curves slightly varied from 35 to 40 cycles in the first PCR reaction and from 15 to 20 cycles in the second PCR reaction, it was confirmed that amplification can reliably be achieved even in the case of a slight DNA amount of a mutant gene.

From the above, it is shown that, when a PCR reaction of the first-primers set is combined, the measurement sensitivity and the reproducibility of the ASP-PCR method are improved by intentionally reducing the concentration of the forward primer for the first PCR and setting the concentration of the primer in the first-primers set having the same direction as the ASP(s) of the second-primers set to 1/100 to 1/20 of the other primer.

### [Example 3]

### Highly-Sensitive Detection of T790M Mutant Allele of EGFR Gene Exon 20 (3)

By using a DNA extracted and purified from the NCI-H1975 cell line as a DNA with T790M mutation of exon 20 of the human EGFR gene and using a DNA extracted and purified from the K562 cell line as a DNA without the mutation, the measurement sensitivity of a measurement system designed according to the present invention was evaluated.

### • Preparation of Template DNA

A template DNA for this example was prepared by mixing a DNA (mutant; mu) extracted from the NCI-H1975 cell line and a DNA (wild) extracted from the K562 cell line in respective proportions so that the total concentration was 30 ng/µL. The wild-derived DNA and the mutant-derived DNA were mixed at mixing ratios (% of mu/wild) of 0.1 %, 0.03 %, 0.01 %, and 0 % (control) for respective preparations. Additionally, the DNA (mutant; mu) extracted from the NCI-H1975 cell line was prepared at 0.003 ng/µL and used as a template having a mixing ratio (% of mu/wild) of 100 %.

### • Reagents

**[Table 4]**

| | |
|---|---|
| 5× Buffer (for Q5) | 5µL |
| 10mM dNTP | 0.5µL |
| 1µM first forward primer (SEQ ID NO:3) | 0.63µL |
| 10µM first/second reverse primer (SEQ ID NO:4) | 1.25µL |
| 10µM second forward primer (SEQ ID NO:5) | 1.25µL |
| 1µM competitive nucleic acid (SEQ ID NO:6) | 2.5µL |
| 20×EvaGreen | 1.25µL |
| 2000U/mL Q5 DNA polymerase | 0.25µL |
| Nuclease-free Water | 7.37µL |
| DNA specimen | 5µL |

### • Amplification Conditions

The conditions were the same as Example 1.

### • Ion Exchange Chromatography Conditions

Anion ion exchange resin column for HPLC: TSKgelDNA-NPR (Tosoh Corporation)
Eluent: 20 mM Tris-HCl (pH 8.6), 0.47 to 0.62 M NaCl gradient (10 min)
Flow rate: 0.75mL/min
Column oven: 25°C
Detector: UV wavelength of 260nm

Fig. 3 shows elution peaks detected when PCR amplification products obtained under the conditions described above were separated by ion exchange chromatography. When the wild-derived DNA was used as the template, no clear elution peak was observed; however, when the mutant-derived DNA (100 %) was used as the template, a main elution peak (arrow) was recognized around 6.3 minutes corresponding to the PCR amplification product, and a similar clear elution peak was confirmed even when the mixing ratio of the mutant-derived DNA was 0.01 %.

From the results described above, it is shown that the mutant gene can be detected with high sensitivity, and the specificity can be ensured as well, by detecting the presence or absence of a specific elution peak obtained by separation by ion exchange chromatography of the PCR amplification product obtained by the measurement system designed according to the present invention.

### [Example 4]

### Quantification of T790M Mutant Allele of EGFR Gene Exon 20 (1)

By using a DNA extracted and purified from the NCI-H1975 cell line as a DNA with T790M mutation of exon 20 of the human EGFR gene and using a DNA extracted and purified from the K562 cell line as a DNA without the mutation, quantitativeness of a measurement system designed according to the invention was evaluated.

### • Preparation of Template DNA

A template DNA of this example was prepared by mixing a DNA (mutant; mu) extracted from the NCI-H1975 cell line and a DNA (wild) extracted from the K562 cell line in respective proportions so that the total concentration was 10 ng/µL. The wild-derived DNA and the mutant-derived DNA were mixed at mixing ratios (% of mu/wild) of 10 %, 3 %, 1 %, 0.3 %, 0.1 %, and 0 % for respective preparations.

### • Reagents

The conditions were the same as Example 3.

### • Amplification Conditions

98°C: 30 seconds
First PCR reaction; 98°C: 10 seconds, 78°C: 15 seconds (55 to 15 cycles)
Second PCR reaction; 98°C: 10 seconds, 56°C: 15 seconds (30 to 47 cycles)

Figs. 4[A] to 4[R] show amplification curves of the second PCR and correlations between a final relative fluorescence intensity (PFU) value and a mutant allele proportion (%) when the amplification reaction was performed by appropriately combining the cycle numbers of the first PCR reaction and the second PCR reaction. If the amplification reaction reaches a saturation phase, the amount of the amplification product generated in the first PCR reaction usually does not reflect the proportion (%) of gene mutation contained in the nucleic acid sample, and therefore, it is considered that the amplification product generated in the second PCR reaction also does not reflect the proportion of gene mutation in the nucleic acid sample. In fact, in [A] to [C] when the cycle number of the first PCR reaction was set to 55 so that the amplification reaction reached a saturation phase, it is seen that the amplification curve of the second PCR reaction does not reflect the proportion (%) of gene mutation contained in the nucleic acid sample at all. In contrast, as in [D] to [F], it is shown that reducing the cycle number of the first PCR reaction to 35 resulted in a condition under which the amplification reaction of the first PCR did not reach a saturation phase, and allowed acquisition, during the subsequent second PCR reaction, of curve amplified from the amplification product by the primers for the first PCR reflecting the amount of gene mutation contained in the nucleic acid sample. Furthermore, by setting the cycle number of the first PCR reaction in the range of 32 to 15 and additionally setting the cycle number of the second PCR reaction to 30 or more, the second PCR reaction using the ASP continued the amplification reaction, while maintaining the specificity, on the amplification product which was obtained by the first PCR reaction and reflecting the amount of gene mutation contained in the nucleic acid sample, so that a more accurate correlation was recognized from the relationship between the PFU value at the final cycle of the second PCR reaction and the proportion (%) of the mutant allele ([G] to [R]).

From the results described above, it is shown that the quantitativeness for the proportion (%) of the mutant allele is maintained by selectively amplifying the mutant gene through the nucleic acid-amplification reaction using the ASP under the condition allowing at least the second-primers set to successively act on the amplification product generated in a manner reflecting an amount of gene mutation contained in the nucleic acid sample before the first PCR reaction reaches a saturation phase.

### [Example 5]

### Quantification of T790M Mutant Allele of EGFR Gene Exon 20 (2)

### • Preparation of Template DNA

The conditions were the same as Example 4.

### • Reagents

The conditions were the same as Example 3.

### • Amplification Conditions

98°C: 30 seconds
First PCR reaction; 98°C: 10 seconds, 78°C: 15 seconds
Second PCR reaction; 98°C: 10 seconds, 56°C: 15 seconds
Cycle number: combination of first PCR + second PCR = 62 cycles

### • Ion Exchange Chromatography Conditions

The conditions were the same as Example 3.

Figs. 5[A], 5[C], 5[E], and 5[G] show elution peaks (with main elution peaks indicated by arrows) obtained by performing a combination of the amplification reactions for the total cycle number of the first and second PCR reactions of 62 and separating and detecting the amplification products with ion exchange chromatography, and Figs. 5[B], 5[D], 5[F], and 5[H] show correlations between the area of the main elution peak and the proportion (%) of the mutant allele.

Under the combined condition in Example 4 of the cycle numbers at which favorable results were obtained in the correlation between the fluorescence intensity after the second PCR reaction and the proportion (%) of the mutant allele, a clear elution peak of the amplification product derived from the mutant allele was recognized around 6.3 minutes, and furthermore, in [A] to [F], favorable correlation was recognized also between the area of the elution peak and the proportion (%) of the mutant allele. Under the condition of 35 cycles of the first PCR reaction in which sufficient quantitativeness was not obtained in Example 4, sufficient results were not obtained in terms of the proportion of the mutant allele although the quantitativeness for the elution peak was recognized.

From the results described above, it is shown that the quantitativeness for the proportion (%) of a mutant allele is ensured by selectively amplifying the mutant gene through a nucleic acid-amplification reaction using an ASP under a condition allowing at least the second-primers set to successively act on an amplification product generated in a manner reflecting the DNA amount of the mutant gene contained in a nucleic acid sample before the first PCR reaction reaches a saturation phase, then separating the amplification product by ion exchange chromatography, and comparing the peak area of the amplification product.

### INDUSTRIAL APPLICABILITY

The method for detecting a mutant gene of the present invention can be used for production of companion diagnostic agent and system for predicting a sensitivity of a tyrosine kinase inhibitor to epidermal growth factor receptor in a cancer patient and production of other companion diagnostic agents and systems serving as a basis for personalized medicine.

## Claims

1. A method for detecting a gene mutation contained in a nucleic acid sample based on the presence or absence of an amplification product from a nucleic acid-amplification reaction using a DNA polymerase, the method comprising the steps of:
in a nucleic acid-amplification reaction solution in which a first-primers set designed to flank a polymorphic site of the gene coexists with a second-primers set containing one or more allele specific primers for selective amplification from a nucleic acid containing the mutation,
(a) obtaining an amplification product by amplifying a nucleic acid containing a mutant allele through a nucleic acid-amplification reaction by the first-primers set, in the presence of a competitive nucleic acid that has a sequence of a wild-type allele of the gene and that hybridizes with all or a portion of the polymorphic site, under a condition preventing a nucleic acid-amplification reaction by the second-primers set; and
(b) causing an amplification reaction with the concentration of a primer(s) of the first-primers set reduced as compared to the concentration of primers of the second-primers set when the amplification product of step (a) is obtained; and
(c) selectively amplifying a nucleic acid containing the mutant allele through a nucleic acid-amplification reaction under a condition allowing at least the second-primers set to act on the amplification product obtained at step (a).

2. The method according to claim 1, wherein the condition preventing a nucleic acid-amplification reaction by the second-primers set is a temperature condition under which the allele specific primer(s) does not anneal to a nucleic acid containing the mutation.

3. The method according to claim 1 or 2, wherein a primer of the second-primers set other than the allele specific primer(s) is designed in common with one primer of the first-primers set.

4. The method according to any one of claims 1 to 3, wherein in the first-primers set, the concentration of a primer having the same direction as the allele specific primer(s) of the second-primers set is 1/100 to 1/20 of the concentration of the other primer(s).

5. The method according to any one of claims 1 to 4, comprising a step of detecting the presence or absence of an amplification product of the second-primers set based on the presence or absence of a peak of the amplification product separated by ion exchange chromatography.

6. A method for quantifying a mutant allele contained in a nucleic acid sample by using an amplification product from a nucleic acid-amplification reaction using a DNA polymerase, the method comprising the steps of:
in a nucleic acid-amplification reaction solution in which a first-primers set designed to flank a polymorphic site of the gene coexists with a second-primers set containing one or more allele specific primers for selective amplification from a nucleic acid containing the mutation,
(a) obtaining an amplification product by amplifying a nucleic acid containing a mutant allele through a nucleic acid-amplification reaction by the first-primers set, in the presence of a competitive nucleic acid that has a sequence of a wild-type allele of the gene and that hybridizes with all or a portion of the polymorphic site, under a condition preventing a nucleic acid-amplification reaction by the second-primers set; and
(b) selectively amplifying, before the nucleic acid-amplification reaction by the first-primers set reaches a saturation phase when the amplification product of step (a) is obtained, a nucleic acid containing the mutant allele through a nucleic acid-amplification reaction under a condition allowing at least the second-primers set to successively act on the amplification product generated in a manner reflecting the DNA amount of the mutant allele contained in the nucleic acid sample.

7. The method according to claim 6, wherein the cycle number of the nucleic acid-amplification reaction by the first-primers set is set to 15 to 32, wherein the cycle number of the nucleic acid-amplification reaction by the second-primers set is set to 30 to 60, and wherein the two reactions are combined and successively performed.

8. The method according to claim 6 or 7, wherein an amplification product of the second-primers set is separated by ion exchange chromatography, and wherein the mutant allele contained in the nucleic acid sample is quantified from a peak area of the amplification product.


SEQUENCE LISTING

<110>  SEKISUI MEDICAL CO., LTD.

<120>  Mutant gene detection method

<130>  17P00907

<160>  6

<170>  PatentIn version 3.5

<210>  1
<211>  360
<212>  DNA
<213>  Homo sapiens

<400>  1
actcaagatc gcattcatgc gtcttcacct ggaaggggtc catgtgcccc tccttctggc      60

caccatgcga agccacactg acgtgcctct ccctccctcc aggaagccta cgtgatggcc     120

agcgtggaca acccccacgt gtgccgcctg ctgggcatct gcctcacctc caccgtgcag     180

ctcatcacgc agctcatgcc cttcggctgc ctcctggact atgtccggga acacaaagac     240

aatattggct cccagtacct gctcaactgg tgtgtgcaga tcgcaaaggt aatcagggaa     300

gggagatacg ggagggggag ataaggagcc aggatcctca catgcggtct gcgctcctgg     360


<210>  2
<211>  360
<212>  DNA
<213>  Homo sapiens


<220>
<221>  mutation
<222>  (188)..(188)

<400>  2
actcaagatc gcattcatgc gtcttcacct ggaaggggtc catgtgcccc tccttctggc      60

caccatgcga agccacactg acgtgcctct ccctccctcc aggaagccta cgtgatggcc     120

agcgtggaca acccccacgt gtgccgcctg ctgggcatct gcctcacctc caccgtgcag     180

ctcatcatgc agctcatgcc cttcggctgc ctcctggact atgtccggga acacaaagac     240

aatattggct cccagtacct gctcaactgg tgtgtgcaga tcgcaaaggt aatcagggaa     300

gggagatacg ggagggggag ataaggagcc aggatcctca catgcggtct gcgctcctgg     360


<210>  3
<211>  19
<212>  DNA
<213>  Artificial Sequence

<220>
<223>  Primer

<400>  3
tgcctcacct ccaccgtgc                                                    19

<210>  4
<211>  22
<212>  DNA
<213>  Artificial Sequence

<220>
<223>  Primer

<400>  4
ctttgtgttc ccggacatag tc                                                22

<210>  5
<211>  16
<212>  DNA
<213>  Artificial Sequence

<220>
<223>  Primer

<400>  5
cgtgcatctc atcttg                                                       16

<210>  6
<211>  20
<212>  DNA
<213>  Artificial Sequence

<220>
<223>  Competitive Oligonucleotide

<400>  6
gctcatcacg cagctcatgc                                                   20